# FASCICULE DE BREVET EUROPEEN

(11) **EP 3 562 554 B1**
(45) Date de publication et mention de la délivrance du brevet: **17.03.2021**
(21) Numéro de dépôt: 17838056.4
(22) Date de dépôt: 26.12.2017
(51) Int. Cl.: A61Q 1/02, A61K 8/04, A61K 8/895, A61K 8/26, A61K 8/41, A61K 8/31

(54) **PRODUIT COSMÉTIQUE SOUS LA FORME DE MOUSSE ET PROCÉDÉ DE MAQUILLAGE.**
KOSMETISCHES PRODUKT IN SCHAUMFORM UND SCHMINKVERFAHREN
COSMETIC PRODUCT IN THE FORM OF A FOAM AND MAKE-UP METHOD

(30) Priorité: 28.12.2016 FR 1663470
(43) Date de publication de la demande: 06.11.2019
(73) Titulaire: LVMH Recherche, 45800 Saint Jean de Braye (FR)
(72) Inventeur: NOE, Brigitte, 45370 Clery Saint André (FR); BICHON, Yohann, 94700 Maisons-Alfort (FR); CHENTOUFI, Naïma, 45800 Saint Jean de Braye (FR)
(74) Mandataire: Cabinet Beau de Loménie
(86) Numéro de dépôt international: PCT/FR2017/053844
(87) Numéro de publication internationale: WO 2018/122527

(56) Documents cités:
- FR-A1- 3 014 312
- US-A1- 2009 269 374
- DATABASE GNPD [Online] MINTEL; 1 mai 2013 (2013-05-01), Ellis Faas: "Creamy Eyes Liquid Eyeshadow", XP002771990, Database accession no. 2054330
- DATABASE GNPD [Online] MINTEL; 1 juillet 2015 (2015-07-01), Deborah: "Watery Mousse Eyeshadow", XP002771991, Database accession no. 3250019
- DATABASE GNPD [Online] MINTEL; 1 juin 2016 (2016-06-01), Johnson & Johnson: "Actif Unify Tinted Mousse Sunscreen SPF 30", XP002771992, Database accession no. 4046365
- DATABASE GNPD [Online] MINTEL; 1 avril 2016 (2016-04-01), Botica Comercial Farmacêutica: "Matte Lip Colour Mousse", XP002771993, Database accession no. 3911397
- DATABASE GNPD [Online] MINTEL; 1 octobre 2016 (2016-10-01), Oriflame: "Matte Mousse", XP002771994, Database accession no. 4374587
- "Formulation Possibilities with new Silicone Elastomer Gel", IP.COM JOURNAL, IP.COM INC., WEST HENRIETTA, NY, US, 18 novembre 2016 (2016-11-18), XP013173630, ISSN: 1533-0001
- Nn: "Gransil PC-12 Product Information", , 1 octobre 2012 (2012-10-01), XP055389790, us Extrait de l'Internet: URL:https://www.ulprospector.com/documents /1030814.pdf?bs=4034&b=115740&st=1&sl=4775 8516&crit=a2V5d29yZDpbZ3JhbnNpbCBwYy0xMl0= &k=gransil|pc-12&r=eu&ind=personalcare [extrait le 2017-07-11]

## Description

La présente invention concerne une composition cosmétique à la texture particulièrement ferme et légère renfermant un gel de silicone, une argile organomodifiée et une grande proportion d'huiles volatiles. Elle se rapporte plus particulièrement au domaine du soin et du maquillage de la peau.

Elle concerne également un procédé cosmétique de maquillage ou de soin comprenant l'application de cette composition sur la peau.

### Art Antérieur

Les produits cosmétiques utilisés comme base de maquillage ou comme fond de teint ont essentiellement pour fonctions le masquage des imperfections de la peau, l'unification du teint, l'obtention d'un aspect plus lisse de la peau et l'amélioration de l'éclat du teint. Pour atteindre ces objectifs, ils contiennent des particules solides tels que des pigments colorés, des composés diffusants, des composés réfléchissants, et des composés matifiants ou floutants.

Les fonds de teint fluides comprennent une dispersion des particules solides dans des liquides tels que l'eau et des huiles pour une application sur la peau plus facile. Augmenter la quantité de particules solides générant des effets optiques, dans ce type d'architecture, permet certes d'améliorer l'efficacité du produit, mais génère deux problèmes. La sensation de légèreté et de confort sur la peau diminue, et il devient difficile de garantir l'homogénéité et la stabilité de la texture. Le formulateur a par conséquent recours à des tensio-actifs, des cires ou des gélifiants, qui ont pour fonction de l'épaissir. Ces composés stabilisants de fonds de teint fluides altèrent cependant les propriétés sensorielles du produit.

Il est également proposé de formuler les particules à effet optique dans une base grasse solide, et non liquide. Les textures solides présentent néanmoins l'inconvénient d'être beaucoup plus difficile à étaler, et des zones inhomogènes de dépôt de produits se forment souvent sur la peau, si bien qu'il est nécessaire de travailler le produit sur la peau assez longuement pour obtenir un maquillage homogène.

Pour remédier à ces divers inconvénients, des textures de consistance intermédiaire entre celle des fonds de teint fluides, présentés en flacon pompe le plus souvent, et celle des fonds de teint crème, présentés en boîtier, ont été proposées.

Certaines de ces textures sont sous forme de mousses, obtenues par introduction d'un gaz en fortes proportions dans une composition fluide à base aqueuse, ce qui permet d'en alléger la texture (le document US 2016/0143842 en est une illustration). Cependant l'introduction d'un gaz rend leur procédé de fabrication plus complexe et diminue la stabilité du produit dans le temps. Ces mousses laissent par ailleurs un film assez lourd sur la peau après application.

Des mousses contenant une combinaison de plusieurs d'élastomères de silicone, notamment des composés de dénomination diméthicone/vinyldiméthicone crosspolymer, lesquels sont par ailleurs utiles pour masquer les rides et lisser la peau, ont également été proposées antérieurement. Ces formules contiennent cependant des huiles hydrocarbonées non volatiles, et leur texture lourde à l'application et laisse un dépôt sur la peau assez présent, peu confortable, ce qui n'est pas satisfaisant quand on cherche au contraire une application légère et couvrante dès les premiers, voire dès le premier, gestes d'étalement.

Le besoin subsiste par conséquent de disposer d'un produit de maquillage ou de soin de la peau permettant de concilier une texture légère et douce ; une application qui ne requiert pas de le travailler excessivement sur la peau ; l'incorporation de grandes quantités de particules solides pour obtenir un masquage efficace des imperfections de la peau ; une bonne stabilité ; et un procédé de fabrication facile à mettre en œuvre et comprenant notamment un nombre d'étapes limité.

Un objectif de l'invention est de s'affranchir de composés formant un film ressenti comme présent sur la peau après application, qui nuisent au confort du produit porté au cours du temps.

Un autre de ses objectifs est de proposer un produit cosmétique dont la texture est proche de celle d'une mousse et dont l'application sur la peau est ressentie comme glissante, légère et non grasse. Le maquillage obtenu avec ce produit doit également permettre une très bonne couvrance des imperfections et laisser sur la peau un fini poudré.

Ces objectifs sont atteints par la combinaison de deux gélifiants d'huiles particuliers, qui permettent d'intégrer des quantités d'huiles volatiles et de particules solides plus élevées que celles pratiquées jusqu'à présent, dans une texture stable, de haute viscosité et très légère à la fois, et dont l'application permet un dépôt homogène dès la première application.

### Description de l'invention

La présente invention a ainsi comme premier objet une composition cosmétique de soin ou de maquillage de la peau selon la revendication 1 comprenant :
- de 35 % à 75%, de préférence de 60 % à 70%, d'au moins une huile volatile,
- de 1% à 6 % d'au moins un polymère de silicone réticulé obtenu par réaction d'un organopolysiloxane comprenant au moins un groupe -Si-H en position terminale avec un organopolysiloxane comprenant au moins deux groupements vinyle, de préférence situés en position terminale le polymère de silicone réticulé étant un polysilicone-11,
- de 0,5% à 5% d'une argile organomodifiée qui est une hectorite organomodifiée modifiée avec un chlorure d'alkylammonium quaternaire à titre d'argile organomodifiée, et
- moins de 5% d'eau,
les pourcentages étant exprimés en masse par rapport à la masse de ladite composition.

L'association spécifique de deux gélifiants d'huile forme une texture innovante, sous la forme d'une mousse, sans qu'il soit nécessaire d'y introduire nécessairement un gaz, ce qui en simplifie le procédé de préparation et permet de s'affranchir de composés structurants qui apportent une sensation lourde ou grasse sur la peau.

Dans la suite du texte les pourcentages sont exprimés en masse par rapport à la masse totale de la composition de l'invention, sauf mention explicite contraire.

### Définitions

Dans la présente demande les expressions « de...à ...» et « entre ... et... » visent à comprendre les bornes inférieure et supérieure de la plage de valeurs. La divulgation d'une plage de valeurs excluant ses bornes vaut divulgation de la plage de valeurs équivalente incluant les bornes, et *vice versa.*

On utilisera indifféremment les termes « polymère de silicone réticulé », « polymère siliconé réticulé » ou « polymère de silicone » dans la présente description.

Une « huile », au sens de la présente invention, pourra être définie comme un composé non soluble dans l'eau (solubilité inférieure à 0,05 mg/L à 20°C), dont le point de fusion, la température de ramollissement ou le point de transition vitreuse à pression atmosphérique est inférieur ou égal à 30°C, de préférence inférieur ou égal à 25°C.

Une huile à caractère volatile pourra être définie par au moins un des critères définis ci-dessous.

La volatilité pourra être définie dans le cadre de l'invention par exemple une pression de vapeur mesurable par une méthode empirique à 25°C, et dont la valeur sera comprise entre 0.13 Pa et 40 000 Pa, par exemple entre 1 Pa et 20 000 Pa, entre 10 Pa et 8 000 Pa, voire entre 15 et 150 Pa. La pression de vapeur sera mesurée selon une des méthodes les mieux adaptées pour le composé d'intérêt, lesquelles méthodes figurent dans les lignes directrices du Test n° 104 de l'OCDE (version 2006). On peut alternativement choisir une huile volatile présentant une température d'ébullition à pression atmosphérique inférieure à 250°C, de préférence inférieure à 230°C et de préférence comprise entre 150°C et 220°C. Enfin, l'huile volatile pourra encore être définie comme une huile ayant un point éclair allant de 35°C to 100°C, de préférence entre 40°C et 80°C.

Par exemple, une huile volatile comme l'isododécane aura une température d'ébullition à 10⁵ Pa comprise entre 175°C et 195°C, un point éclair de 45°C et une pression de vapeur à 20°C égale à 100 Pa. Sa solubilité dans l'eau à 20°C est inférieure ou égale à 1.0 10⁻² mg/L.

Une autre huile volatile comme la cyclopentadiméthylsiloxane a une solubilité dans l'eau à 25°C égale à 1.7 10⁻² mg/L, un point éclair de 77°C, un point d'ébullition à 10⁵ Pa égal à 205°C, et une pression de vapeur égale à 26 Pa à 25°C.

### Polymère de silicone réticulé

Le polymère de silicone réticulé est gélifié dans la composition par un solvant dudit polymère. On préfère qu'il soit sous la forme de particules gélifiées dont la taille moyenne est comprise entre 10 et 200 microns. Le polymère de silicone réticulé peut être désigné comme étant un élastomère de silicone par l'homme du métier.

On utilise indifféremment dans la présente description les expressions « polymère de silicone réticulé gélifié ou non gélifié » et « particules de polymère de silicone réticulé gélifiées ou non gélifiées ». On sous-entend que « gélifié(e) » désigne « gélifié(e) » par un solvant du polymère. L'expression « polymère de silicone réticulé » pourra désigner le polymère de siliconé réticulé gélifié ou non gélifié.

Le polymère de silicone réticulé peut être obtenu par réaction d'hydrosilylation des deux organopolysiloxanes mentionnés précédemment, en présence d'un catalyseur et d'une huile, dans des conditions de réaction connues de l'homme de l'art. Le catalyseur peut être l'acide hexachloroplatinique, ou un complexe de platine.

On pourra alternativement utiliser un polymère de silicone réticulé obtenu par réaction de condensation réticulation déshydrogénation entre les deux organopolysiloxanes décrits précédemment, en présence d'un catalyseur et d'une huile.

L'huile utilisée pour la préparation du polymère de silicone réticulé peut être une huile non volatile, ou une huile volatile identique à une des huiles volatiles décrites ci-après entrant dans la composition du produit de l'invention. On citera l'isododécane ou la décaméthylcyclopentasiloxane comme solvant.

On préfère que l'une au moins des organopolysiloxanes mentionnées précédemment comprennent majoritairement des motifs diméthylsiloxanes, les autres motifs pouvant être méthylphénylsiloxane ou diméthylvinylsiloxy pour un organosiloxane vinylique, et méthylhydrogénosiloxane pour l'organopolysiloxane contenant des groupes -Si-H.

L'organopolysiloxane comprenant au moins un groupement à insaturation éthylénique lié à un atome de silicium peut être choisi parmi les copolymères dont une partie des motifs comprennent des groupements vinyles ou pour lesquels au moins un groupement vinyle est en bout de chaîne.

L'organopolysiloxane comprenant au moins un groupement vinyle peut être choisi parmi les copolymères méthylvinylsiloxane/diméthylsiloxane, les polydiméthylsiloxanes à terminaisons diméthylvinylsiloxy, les copolymères diméthylsiloxane/méthylphénylsiloxane à terminaisons diméthylvinylsiloxy, et les copolymères diméthylsiloxane/méthylvinylsiloxane à terminaisons triméthylsiloxy.

L'organopolysiloxane contenant des groupes -Si-H et l'organopolysiloxane comprenant au moins deux groupements vinyle terminaux sont de préférence utilisés dans des proportions telles que le ratio molaire entre la quantité totale d'atomes d'hydrogène liés à des atomes de silicium et la quantité totale des groupements vinyle est compris entre 1,5/1 et 20/1.

L'organopolysiloxane contenant des groupes -Si-H peut être une polydiméthylsiloxane ou une poly(diméthyl)(méthylhydrogéno)siloxane, l'une ou l'autre comprenant au moins une liaison Si-H en bout de chaîne, de préférence deux liaisons Si-H en bout de chaîne.

Ainsi, les copolymères réticulés obtenus à partir d'un organopolysiloxane ne contenant pas des groupes -Si-H en bout de chaîne tels que les copolymères triméthylsilyl terminated methylhydrosiloxane dimethylsiloxane dont un nom INCI peut être Dimethicone/Vinyldimethicone Crosspolymer et qui sont venus par exemple sous la marque KSG®, ne font pas partie de la présente invention. Les inventeurs ont montré que ces polymères ne conduisent à un produit épais, brillant et instable.

Le polymère de silicone réticulé est par exemple le produit de réaction d'une polydiméthylsiloxane ou d'une poly(diméthyl)(méthylhydrogéno)siloxane, comprenant l'une ou l'autre au moins une liaison Si-H terminale, avec une polydiméthylsiloxane comprenant deux groupements vinyles (plus précisément vinyldiméthyles), de préférence située en position terminale de la chaîne.Un tel polymère est disponible sous la marque Gransil®, par exemple le produit Gransil® PC-12.

On préfère que le deuxième organopolysiloxane ne comprenne pas de motifs vinylméthylsiloxane, de manière à ce que le polymère ne comprenne que deux groupements vinyl situés en bout de chaîne.

Le polymère siliconé réticulé est sous forme d'un gel dans la composition.

Par exemple, le gel de polymère siliconé comprend des particules de polymère siliconé réticulé gélifiées emprisonnant des molécules d'un solvant, lequel solvant représente entre 10 et 95% en masse de la masse du gel. La proportion de solvant contenu dans le gel peut varier de 60 % à 95% en poids, par exemple de 80 % à 90% en poids. Un tel gel de polymère siliconé réticulé peut être fabriqué en appliquant un fort cisaillement à des particules de polymère siliconé réticulé (qui ont été préalablement synthétisées à partir des deux organopolysiloxanes décrites précédemment), ledit cisaillement étant exercé en présence d'un solvant du polymère de silicone réticulé. Le cisaillement peut être réalisé dans un homogénéisateur à haute pression, de manière à obtenir des particules de polymères gélifiées par ledit solvant ; leur taille peut varier entre 10 microns et 200 microns. On parlera indifféremment d'un gel de polymère siliconé réticulé dans un solvant ou de particules de polymère siliconé réticulé gélifiée par un solvant.

Le solvant qui gélifie les particules de polymère siliconé réticulé peut être une huile non volatile, ou de préférence une huile volatile choisie parmi les huiles volatiles hydrocarbonées, les huiles volatiles siliconées ou un de leurs mélanges, ces huiles étant conformes à la description des huiles volatiles donnée plus loin.

Le polymère de silicone réticulé entrant dans la composition de l'invention porte le nom INCI POLYSILICONE-11. On préfère utiliser du POLYSILICONE-11 qui est sous forme gélifié par l'isododécane, avant son mélange avec les autres ingrédients de la composition, et/ou dans la composition ainsi obtenue.

L'homme du métier pourra vérifier par des méthodes conventionnelles que le polymère de silicone réticulé est sous forme d'un gel dans la composition.

Lorsque le polymère de silicone réticulé est sous la forme d'un gel, la quantité de polymère de silicone réticulé présent dans la composition peut être exprimée comme la quantité de polymère équivalente, sous-entendu qui ne serait pas gélifiée en l'absence du solvant contenu dans le gel. Alternativement, la quantité de polymère de silicone réticulé présent dans la composition peut correspondre à la quantité du gel dans la composition.

La quantité de polymère équivalente du polymère gélifié va de 1 à 6% en poids de polymère siliconé réticulé par rapport au poids de la composition de soin ou de maquillage de l'invention. Ce pourcentage est équivalent au pourcentage de polymère siliconé réticulé s'il était non gélifié dans la composition. La quantité équivalente de polymère siliconé réticulé non gélifié est de préférence comprise entre deux valeurs choisies dans le groupe constitué par 1%, 1,5%, 2%, 2,5%, 3%, 3,5%, 4%, 4,5%, 5%, 5,5% et 6%.

La quantité du gel contenant le polymère de silicone réticulé gélifié par un solvant est de préférence comprise entre deux valeurs choisies dans le groupe constitué par 20%, 22%, 24%, 26%, 28%, 30%, 32%, 34%, 36%, 38%, 40%, 42%, 44%, 46%, 48% et 50%.

### Argile Organomodifiée

La composition de l'invention contient de 0,5 à 5% en masse d'une argile organomodifiée. L'argile organomodifiée est une hectorite organomodifiée modifiée avec un chlorure d'alkylammonium quaternaire, de préférence un ammonium substitué par au moins un, de préférence au moins deux alkyles comprenant de 14 à 20 atomes de carbone. L'alkyle peut être le stéaryle. On citera le composé de nom INCI disteardimonium hectorite dans lequel l'ammonium comprend deux méthyles et deux stéaryles.

La proportion d'argile modifiée peut être comprise entre deux valeurs choisies dans le groupe constitué par 0,5%, 1%, 1,5%, 2%, 2,5%, 3%, 3,5%, 4%, 4,5%, et 5%.

Selon un mode de réalisation, la composition comprend de 1% à 3% en masse d'une hectorite modifiée avec un chlorure de stéaryl ammonium quaternaire.

### Volatiles

Les huiles volatiles qui sont utilisées dans la composition de l'invention sont de préférence des silicones ou des hydrocarbures saturés à chaîne ramifiée.

L'huile volatile peut notamment être choisie parmi les huiles siliconées telles que les diméthicones (polydiméthylsiloxanes) dont la viscosité va de 0,5 à 6 cSt et les cyclodiméthicones.

L'huile volatile peut être le néopentanoate d'iso-hexyle ou un hydrocarbure tel que l'isododécane, l'isodécane, l'isohexadécane, le n-dodécane (C₁₂) et le n-tétradécane (C₁₄) ou le mélange undécane-tridécane.

On mentionnera par exemple l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

Une huile volatile peut être l'isododécane.

Selon un mode de réalisation particulier de l'invention la composition de l'invention contient au moins deux huiles volatiles choisies parmi les alcanes et les cyclométhicones, par exemple la décaméthylcyclopentasiloxane et l'isododécane.

On préfère que l'isododécane soit majoritaire dans un mélange d'huiles volatiles, ou qu'il soit l'unique huile volatile de la composition.

Dans un mode de réalisation particulier, les huiles volatiles contenues dans la composition sont constituées de préférence par un mélange d'isododécane et de décaméthylcyclopentasiloxane dans un ratio massique compris entre 1/1 et 3/1, par exemple de l'ordre de 2/1.

On pourra également utiliser dans le cadre de la présente invention d'autres huiles volatiles telles que celles décrites dans la demande FR2873581.

La proportion des huiles volatiles dans la composition est de préférence comprise entre 35 et 75%, de préférence encore entre 60% et 70% en masse par rapport à la masse de la composition. La proportion des huiles volatiles peut être comprise entre deux valeurs choisies dans le groupe constitué par 35%, 40%, 45%, 50%, 55%, 60%, 65%, 70% et 75%.

### Viscosité, Densité et Stabilité

La viscosité de la composition à 25°C et pression atmosphérique est comprise entre 50 000 mPa.s et 500 000 mPa.s, de préférence comprise entre 80 000 mPa.s et 300 000 mPa.s.

Cette viscosité peut être mesurée avec un viscosimètre Rhéolab QC (Anton Paar) doté du logiciel Rheoplus en utilisant un mobile et un temps de mesure adaptés, par exemple dans les conditions suivantes:

| Géométries | Vitesse de rotation (tr/min) | Temps de mesure (min) |
|---|---|---|
| Cylindre coaxial : CC27 | 200 | 3 |
| 4 Pales : ST22 - 4V | 100 | 3 |
| Ailette : ST24-2D-2V-2V | 50 | 3 |
| Ancre : ST22 - 2V | 10 | 7 |

Préalablement à la mesure, on aura placé la composition de l'invention dans un pot de 120 ml (Kola Rond VT3 M120 Blanc Pharm) dans une étuve à 25°C pendant 12 heures minimum. Une fois le mobile plongé dans le pot, le niveau de composition doit atteindre le col du pot.

On vérifie que le mobile est bien choisi en mesurant le pourcentage de déviation des mesures qui sont effectuées toutes les 6 secondes. La valeur de la viscosité de La composition est égale, selon ce protocole, à la moyenne des quinze dernières mesures effectuées par l'appareil pendant le temps de mesure indiqué ci-dessus.

La densité de la composition à 25°C et pression atmosphérique est comprise entre 0,8 g/cm³ et 1,2 g/cm³, par exemple de 0,85 g/cm³ à 1,15 g/cm³ et de préférence entre 0,9 g/cm³ et 1 g/cm³. Elle peut être mesurée selon une méthode connue de l'homme du métier.

La composition de l'invention est avantageusement stable, en ce sens qu'elle ne décante pas au cours du temps et conserve sa texture légère. La stabilité peut ainsi être vérifiée par un examen visuel après stockage de La composition dans une étuve à 50°C, 45°C ou 4°C pendant 1 ou 2 mois.

La composition est de préférence dotée cumulativement des propriétés suivantes : stable avec une viscosité à 25°C et pression atmosphérique comprise entre 50 000 mPa.s et 350 000 mPa.s, et une densité à 25°C et pression atmosphérique comprise entre 0,8 g/cm³ et 1,2 g/cm³.

Selon un mode de réalisation de l'invention, la composition a une viscosité à 25°C et pression atmosphérique comprise entre 200 000 mPa.s et 250 000 mPas.s, une densité à 25°C et pression atmosphérique comprise entre 0,9 g/cm³ et 1,0 g/cm³ et comprend, outre le polymère de silicone réticulé et l'argile organomodifiée, de 35% à 75%, de préférence de 60% à 70% en masse d'au moins une huile volatile, et de 10 à 30% en masse de particules solides.

Le mélange des huiles contenues dans la composition, notamment les huiles volatiles et éventuellement les huiles non volatiles, a de préférence une densité à 25°C et pression atmosphérique inférieure à 0,9 g/cm³, de préférence inférieure à 0,85 g/cm³, voire même inférieure à 0,8 g/cm³ et supérieure à 0,7 g/cm³.

### Combinaison polymère siliconé et argile

Le ratio massique entre le polymère de silicone réticulé non gélifié, le polysilicone-11, et l'argile organomodifiée, l'hectorite modifiée est compris entre 1/1 et 10/1.

En particulier on préfère une composition présentant au moins une des caractéristiques suivantes, par exemple une, deux ou trois de caractéristiques suivantes :
- un ratio massique entre le polymère de silicone réticulé non gélifié et l'argile organomodifiée compris entre deux valeurs choisies dans le groupe constitué par 1/1, 2/1, 3/1, 4/1, 5/1, 6/1, 7/1, 8/1, 9/1 et 10/1. Par exemple, le ratio massique est supérieur ou égal à 18/10, 19/10, 2/1, 21/10, 22/10, 23/10, et 24/10, et 25/10.
- une quantité de polymère de silicone réticulé (par rapport à la masse équivalente du polymère dans son état non gélifié) comprise entre deux valeurs choisies dans le groupe constitué par 1%, 1,5%, 2%, 2,5%, 3%, 3,5%, 4%, 4,5%, 5%, 5,5% et 6%,
- une quantité de polymère de silicone réticulé gélifié avec un solvant, de préférence une huile volatile citée précédemment, comprise entre deux valeurs choisies dans le groupe constitué par 20%, 22%, 24%, 26%, 28%, 30%, 32%, 34%, 36%, 38%, 40%, 42%, 44%, 46%, 48% et 50%,
- une quantité d'argile modifiée comprise entre deux valeurs choisies dans le groupe constitué par 0,5%, 1%, 1,5%, 2%, 2,5%, 3%, 3,5%, 4%, 4,5%, et 5%,
les pourcentages étant exprimés en masse par rapport à la masse de la composition.

En particulier, la demande décrit une composition dont la viscosité dynamique à 25°C et pression atmosphérique est de préférence comprise entre 50 000 mPa.S et 350 000 mPa.s contenant de 35% à 75 % en masse d'au moins une huile volatile, de 1% à 6% en masse de polysilicone-11, de 0,5% à 5% en masse d'une hectorite modifiée, et moins de 5% en masse d'eau.

### Particules solides

On peut avantageusement incorporer dans le mélange d'huile volatile, de polymère siliconé réticulé et d'argile organomodifiée tels qu'ils sont décrits précédemment, des particules solides tels que des pigments et des charges de manière à obtenir des effets optiques, telles que la couleur, la réflexion de la lumière ou la diffusion de la lumière. Une charge par opposition à un pigment est incolore.

La composition de l'invention contient avantageusement des particules solides dotées de propriétés optiques particulières.

Les particules solides ayant un effet optique, tel que défini dans la présente invention, peuvent être des particules qui absorbent la lumière visible, produisant ainsi un effet de couleur, tels que des pigments et nacres, des particules ayant une grande réflectance diffuse, une faible réflexion spéculaire et une haute transmittance diffuse, appelées couramment des particules soft-focus, des particules floutantes ou matifiantes qui réduisent la brillance en absorbant le sébum.

Parmi les pigments minéraux, on peut citer, à titre d'exemples le dioxyde de titane, éventuellement traité en surface; les oxydes de fer noir, jaune, rouge et brun; le violet de manganèse; le bleu outremer; l'oxyde de chrome; l'oxyde de chrome hydraté ; le noir de carbone et le bleu ferrique.

Parmi les pigments organiques, on peut citer, par exemple, les pigments D & C Red n° 19; D & C Red n° 9; D & C Red n° 21; D & C orange n° 4; D & C orange n° 5; D & C Red n° 27 ; D & C Red n° 13 ; D & C Red n° 7 ; D & C Red n° 6 ; D &C Yellow n° 5 ; D & C Red n° 36 ; D & C orange n° 10 ; D & C Yellow n° 6 ; D & C Red n° 30 ; D &C Red n° 3.

On pourra également utiliser des pigments interférentiels, tels que des nacres, capables de produire une couleur par un phénomène d'interférence, par réflexion de la lumière par des couches superposées de matériaux d'indices de réfraction différents. Les pigments nacrés peuvent être choisis notamment parmi les pigments nacrés blancs, tels que le mica recouvert d'oxyde de titane, l'oxychlorure de bismuth; et les pigments nacrés colorés, tels que le mica titane avec des oxydes de fer, le mica titane avec du bleu ferrique ou de l'oxyde de chrome, le mica titane avec un pigment organique du type précité, ainsi que les pigments à base d'oxychlorure de bismuth.

Des exemples de poudres à effet floutant et/ou matifiant (encore appelées charges) comprennent des poudres d'alumine, de silice, de silicate d'aluminium, de silice pyrogénée, de silylate de silice, de dioxyde de titane ou de séricite. Les charges de granulométrie inférieure à 20 microns, et de nature organique telles que les poudres composites de talc/dioxyde de titane/alumine/silice, les poudres de polyamide (Nylon 12), le poly(méth)acrylate de méthyl (sphères creuses de PMMA), des copolymères acrylate/styrène, le hexyldecyldiisocyanate triméthylol hexyl lactone crosspolymer, le PTFE micronisé, la fluorphlogopite synthétique et le nitrure de bore, peuvent être utilisées dans le cadre de l'invention.

On préfère dans le cadre de l'invention ne pas utiliser des particules présentant des groupements -OH, telles que la silice non traitée par exemple. On préférera utiliser par conséquent des silices pour lesquelles les groupes -OH de surface ont été modifiés telles que la silice diméthyl silylate et la silylate de silice.

Les particules solides sont avantageusement contenues dans la composition de l'invention en des quantités comprises entre 10 et 30% en masse, par exemple entre 15% et 25% en masse. Le rapport massique entre les particules solides et le polymère de siliconé réticulé est de préférence compris entre 4/1 et 6/1. Il est par exemple de l'ordre de 5/1.

La composition peut comprendre de 3% à 12% en masse de charges et de 8% à 15% de pigments.

### Ingrédients additionnels

On pourra incorporer dans la composition de l'invention une quantité de polymère qui confère des propriétés filmogènes à un dépôt de la composition sur la peau, et destiné à augmenter la tenue du film de composition sur la peau. La quantité de polymère va par exemple de 1% à 10% en masse, de préférence encore de 3% à 7%, voire même de 4% à 6% en par rapport à la masse de la composition.

Selon un mode de réalisation de l'invention, on utilise une résine siliconée comme polymère filmogène.

Une composition selon l'invention peut comprendre à titre de polymère filmogène au moins une résine siliconée choisie dans le groupe constitué des résines siliconées comprenant au moins une unité motifs choisi parmi les unités M (trialkylSiO_{1/2}), D (dialkylSiO_{2/2}), T (alkylSiO_{3/2}) et Q (SiO_{4/2}).

Comme exemple de résines siliconées solides de type MQ on peut citer les triméthylsiloxysilicates et les phénylalkylsiloxysilicates telle que la phénylpropyldiméthylsiloxysilicate.

La composition peut contenir de 1% à 10% en masse d'une résine siliconée, de préférence de 1% à 10% en masse d'une résine triméthylsiloxysilicate par rapport à la masse de ladite composition.

Des résines siliconées comprenant des motifs T et éventuellement D, peuvent être des polysilsesquioxanes comprenant éventuellement des groupes terminaux Si-OH. De préférence, on peut utiliser les résines polyméthylsilsesquioxanes.

On peut mentionner également les résines comprenant des motifs M, Q et T telles que les résines MQT-propyl.

On pourra incorporer tout autre polymère filmogène connu de l'homme du métier qui soit soluble dans l'huile volatile ou le mélange des huiles volatiles décrites plus haut.

La composition pourra comprendre de 1% à 15% en masse, ou de 1% à 10% en masse, d'une ou plusieurs huiles non volatiles siliconées ou hydrocarbonées, dont la fonction première est de disperser les particules solides, notamment les pigments, lorsque la composition en contient.

Les huiles siliconées peuvent être choisies parmi les huiles siliconées phénylées, telles que par exemple les phényl triméthicones, les phényl diméthicones, les phényl triméthylsiloxy les diphénylsiloxanes, les diphényl diméthicones, les diphényl méthyldiphényl trisiloxanes.

L'huile non volatile siliconée utilisable dans l'invention peut notamment être choisie parmi les polydiméthylsiloxanes (PDMS) de viscosité supérieure à 10 centistokes (cSt), les PDMS comportant des groupements alkyle, hydroxyle ou alcoxy et les polyalkylméthylsiloxanes.

La composition pourra également comprendre une huile hydrocarbonée non volatile connue de l'homme du métier, bien que l'on préfère s'en affranchir dans la cadre de la présente invention.

On préfère que la composition contienne 1% à 15% en masse d'une huile non volatile siliconée, telle qu'une phényl triméthicone.

Outre les ingrédients décrits précédemment, la composition de l'invention peut comprendre au moins un excipient cosmétiquement acceptable qui peut être choisi parmi des parfums, des agents biologiquement actifs destinés à améliorer l'apparence de la peau, des gélifiants d'huile, des colorants, des édulcorants pour masquer l'amertume de certains composés et des conservateurs.

A titre d'exemples de composés gélifiants d'huile pouvant être utilisés dans le cadre de l'invention et qui augmentent la viscosité d'une des huiles citées précédemment, on peut citer les micas naturels modifiés tel que le fluorosilicate d'aluminium, de magnésium et de potassium; les argiles organomodifiées qui sont des argiles traitées par des composés choisis notamment parmi les amines quaternaires, les amines tertiaires; les silices pyrogénées traitées en surface par un composé siliconé, et les éthylcelluloses.

La composition selon l'un des aspects de l'invention peut répondre à une des caractéristiques suivantes ou à une combinaison de plusieurs de ces caractéristiques :
- comprendre moins de 2% en poids, de préférence moins de 0,5% en poids, de préférence encore moins de 0,1% voire être exclue d'aérogel de silice (nom INCI : Silica silylate) tel que décrit notamment dans le brevet US 7470725. Ces aérogels sont des matériaux poreux obtenus en remplaçant l'huile contenu dans un gel de silice par de l'air. Les aérogels contiennent donc une grande proportion de gaz. Or les inventeurs ont trouvé qu'il est possible d'obtenir une composition cosmétique ayant la texture reproduisant celle d'une mousse sans qu'il soit nécessaire qu'elle contienne un gaz, ce qui facilite considérablement le procédé de fabrication.
- être obtenue selon un procédé de fabrication et de conditionnement dans un récipient, lequel procédé ne comprend pas nécessairement une étape d'injection d'un gaz après mélange des ingrédients mentionnés plus haut, le gaz pouvant représenter entre 30% et 65% en volume du volume de produit cosmétique dans lequel on souhaite injecter un gaz. Un tel gaz est par exemple choisi parmi l'argon, l'hélium, le diazote, l'oxyde d'azote, le dioxygène, le dioxyde de carbone, le butane ou le propane. De préférence, le procédé de préparation de la composition de l'invention ne comprend pas une étape d'injection de gaz.
- comprendre moins de 5% en poids de composés ayant de groupements OH libres telle que la silice par exemple,
- comprendre moins de 1% en masse, de préférence moins de 0,5%, voire même être dépourvue de composé(s) gras solides ou de composé(s) gras comprenant une phase solide, tels que des cires ou des composés pâteux. On citera notamment comme composé gras solide, l'acide stéarique, une cire de polyéthylène, une cire synthétique, une cire de paraffine ou une cire microcristalline, l'alcool cétylique, l'alcool stéarylique, l'alcool béhénylique et le stéarate de glycéryle. Ces composés peuvent en effet augmenter la sensation de collant et diminuer la sensation de légèreté que le film de composition génère sur la peau.
- comprendre moins de 15% en masse, de préférence moins de 10%, voire moins de 2% en masse, de préférence être dépourvue d'huiles hydrocarbonées non volatiles. On pourra également citer les esters aliphatiques tels les esters de faible masse moléculaire comprenant de 10 à 40 atomes de carbone, par exemple l'isononanoate d'isonoyle, les monoesters d'acides gras comme le butyl isostearate, les alcools gras tels que le 2-octyldodécanol. Parmi les huiles hydrocarbonées, on peut également citer les huiles constituées de carbone et d'hydrogène comme le polyisobutène hydrogéné, l'huile de paraffine et le squalane.
- comprendre moins de 0,5% en masse de filtres solaires organiques, car ces derniers diminuent considérablement les qualités sensorielles de la composition.

La composition de l'invention selon un des aspects décrits précédemment, peut servir de base à un produit de maquillage de la peau du visage ou des paupières. La composition de l'invention peut se présenter sous la forme d'un produit de maquillage de la peau du visage (fond de teint), ou d'un produit de maquillage des paupières (fard à paupières).

Elle peut également servir de base à un produit de soin de la peau du visage, du cou ou du contour des yeux (lotion, sérum, crème), ou de base de maquillage pour masquer les imperfections de la peau ou véhiculer des actifs.

Selon un deuxième aspect, l'invention concerne un procédé de soin ou de maquillage de la peau qui consiste à appliquer sur la peau du visage, du cou ou des paupières, une composition telle que décrite précédemment.

Toutes les caractéristiques qui ont été décrites en rapport avec ces compositions s'appliquent au procédé de maquillage de l'invention.

Dans un mode de réalisation, le procédé comprend une étape de préhension de la composition par un utilisateur, la préhension étant de préférence effectuée avec les doigts ou la main. L'étape de préhension a pour avantage de ne pas détruire la texture de la composition lorsqu'elle est déposée sur la peau. Dans une étape ultérieure, l'utilisateur étale la mousse déposée sur la peau à l'aide des doigts, de la main, ou d'un moyen d'application. La composition qui avait été déposée sous la forme d'une mousse par exemple, se transforme alors en un film mat à l'effet poudré qui recouvre la peau sous l'effet du geste d'étalement.

La fraction de composition prélevée du récipient par l'utilisateur peut avantageusement présenter la texture d'une mousse pendant la préhension, et pendant le dépôt de la fraction préalable à l'étalement sur la peau. En étalant la mousse déposée sur la peau, la composition change de texture et forme un film essentiellement dépourvue d'une huile qui recouvre la zone de peau concernée. Par essentiellement dépourvue on entend une teneur inférieure à 15%, de préférence inférieure à 12%.

### Procédé de préparation

Enfin, il est décrit un procédé de fabrication d'une des compositions décrites précédemment.

Selon un mode de réalisation préférentiel, le polymère de silicone réticulé et/ou l'argile organomodifiée sont fournis sous forme d'un gel (ou d'une dispersion) dans une huile, avant d'être mélangés aux autres composés entrant dans la composition du produit de l'invention.

Aussi, selon un mode de réalisation particulier, le procédé de fabrication est caractérisé en ce qu'il comprend une étape de préparation ou de mise à disposition d'un gel de polymère de silicone dans une huile volatile, de préférence l'isododécane, ainsi qu'une étape de fabrication ou de mise à disposition d'un gel d'argile organomodifiée dans une huile volatile, de préférence l'isododécane.

Par exemple, le gel de polymère siliconé comprend des particules de polymère siliconé réticulé gélifiées emprisonnant des molécules d'un solvant, lequel solvant peut représenter entre 10 et 95% en masse de la masse du gel. La proportion de solvant contenu dans le gel peut varier de 60 à 95% en poids, par exemple de 80 à 90% en poids. Le gel de polymère siliconé peut être fabriqué en appliquant un fort cisaillement à des particules de polymère siliconé réticulé en présence d'un solvant du polymère. Le cisaillement peut être réalisé dans un homogénéisateur à haute pression, de manière à obtenir des particules de polymères gélifiées dont la taille varie entre 10 microns et 200 microns.

On parlera indifféremment d'un gel de polymère siliconé réticulé dans un solvant ou de particules de polymère siliconé réticulé gélifiées par un solvant.

Le solvant du polymère qui permet de la gélifier est de préférence une huile volatile décrite précédemment, par exemple l'isododécane.

Dans un mode de réalisation particulier, le procédé décrit comprendra une étape de mise à disposition d'un gel de polymère siliconé réticulé dans une huile volatile, une étape de mise à disposition d'un gel d'argile organomodifiée dans une huile volatile, et une étape de mélange du gel de polymère siliconé réticulé et du gel d'argile organomodifiée de manière à former un pré-mélange. Dans une étape ultérieure aux précédentes, le pré-mélange pourra être éventuellement mélangé à une quantité d'une ou plusieurs huiles volatiles pouvant être identiques au solvant du polymère ou au solvant contenu dans le gel d'argile organomodifiée, qui ont été utilisés pour préparer le pré-mélange.

Le pré-mélange pourra également être mélangé, le cas échéant, à d'autres ingrédients, tels que des particules solides, des polymères filmogènes et des huiles non. Des particules solides telles que des pigments ou des charges à effet optique peuvent être ajoutées pour préparer une composition destinée au soin ou au maquillage de la peau.

Dans une étape finale, la composition cosmétique constituée des ingrédients décrits ci-dessus, pourra être conditionnée dans un récipient muni d'un moyen de fermeture tel qu'un pot cylindrique ou un tube.

Le procédé décrit permet d'obtenir un produit dont la texture est analogue à celle d'une mousse, sans mettre nécessairement en œuvre une étape d'injection de gaz après l'étape de mélange des ingrédients, et avant l'étape de conditionnement de la composition dans un récipient.

Il est décrit par ailleurs un produit de fond de teint pour le maquillage du visage, qui est conditionné dans un flacon ou dans un tube doté d'un orifice suffisamment large pour ne pas détruire la texture de la mousse au moment de la sortie du produit. Un conditionnement comprenant un moyen d'application solidaire d'un contenant tel qu'un pinceau ou un crayon n'est pas adapté à la distribution du produit, car il risquerait de fluidifier la mousse avant son étalement sur la peau par l'utilisateur.

L'invention est illustrée plus en détail par les exemples suivants. Le mélange de tous les ingrédients est réalisé de préférence sous agitation, à pression atmosphérique et à basse température, par exemple à 30°C, 25°C ou 20°C.

### Exemples:

On a préparé les produits dont la composition massique est indiquée dans le Tableau 1.

**Tableau 1**

| | Exemple 1 | Exemple comparatif 1 | Exemple comparatif 2 |
|---|---|---|---|
| Nom INCI ou nom chimique | %en masse | | |
| POLYSILICONE-11 (GRANSIL® PC-12 ) | 3,6 | 4,9 | - |
| DIMETHICONE/VINYLDIMETHICONE CROSSPOLYMER (KSG-16 ®) | - | - | 3,6 |
| DISTEARDIMONIUM HERCTORITE | 1,3 | - | 1,3 |
| PROPYLENE CARBONATE | 0,4 | - | 0,4 |
| TRIMETHYLSILOXYSILICATE | 2,0 | 2,0 | 2,0 |
| PHENYL TRIMETHICONE | 10,0 | 10,0 | 10,0 |
| METHYL METHACRYLATE CROSSPOLYMER | 6,1 | 6,1 | 6,1 |
| SYNTHETIC FLUORPHLOGOPITE | 2,4 | 2,4 | 2,4 |
| Pigments | 10,0 | 10,0 | 10,0 |
| Solvants volatils | Qsp 100 | Qsp 100 | Qsp 100 |

### • Procédé de fabrication

Dans le bécher principal, on disperse l'hectorite organomodifiée dans les solvants volatils, sous Rayneri. Quand la phase est homogène, on y ajoute le propylène carbonate jusqu'à obtention d'un gel homogène.

On ajoute ensuite le polysilicone-11 ou le Dimethicone/vinyldimethicone Crosspolymer à ce mélange. On homogénéise le mélange sous Rayneri jusqu'à ce qu'il soit visuellement homogène.

Dans un bécher secondaire, on disperse au tricylindre les pigments dans la phényltriméthicone (3 passages de tricylindre). On ajoute cette dispersion de pigments dans le bécher principal, puis on homogénéise le mélange sous Rayneri jusqu'à ce qu'il soit visuellement homogène.

On ajoute les autres ingrédients, et on mélange sous Rayneri jusqu'à ce que le tout soit visuellement homogène.

### • Etude de stabilité

Les compositions de fond de teint de chacun des exemples sont placées en étuve à 25 °C pendant 24 heures.

Résultats : Les résultats sont présentés dans le tableau 2 ci-dessous. Le produit de l'exemple 1 selon l'invention est stable, tandis que les produits des exemples comparatifs 1 et 2 sont déphasés.

### • Mesure de la viscosité et texture

La viscosité de l'exemple 1 est mesurée avec un viscosimètre Rhéolab QC (Anton Paar) doté du logiciel Rheoplus.

La viscosité des exemples comparatifs 1 et 2 est mesurée avec le mobile Ailette tournant à 50 tour/min pendant 3 minutes, tandis que la viscosité de l'Exemple 1 selon l'invention est mesurée avec le mobile Ancre (ST22-2V) tournant à 10 tour/min pendant 7 minutes.

Préalablement à la mesure, chaque composition est versée dans un pot de 120 ml (Réf : 102171001, Kola Rond VT3 M120 Blanc Pharm) puis placée dans une étuve à 25°C pendant 12 heures minimum. Une fois le mobile plongé dans le pot, le niveau de composition atteint le col du pot.

La valeur de la viscosité est égale à la moyenne des quinze dernières mesures effectuées par l'appareil pendant le temps de mesure indiqué ci-dessus.

Résultats : Les résultats sont présentés dans le tableau 2 ci-dessous. La composition de l'invention a la texture d'une mousse à l'aspect granité en surface, qui ne s'écoule pas lorsqu'on retourne le pot, tandis que la composition de l'exemple comparatif 1 et de l'exemple comparatif 2 ont la texture d'un fluide épais qui s'étale assez rapidement sur une surface plane.

**Tableau 2**

| | Exemple 1 | Exemple comparatif 1 | Exemple comparatif 2 |
|---|---|---|---|
| Aspects | Mousse ferme mate | Fluide épais brillant | Fluide épais brillant |
| Viscosités mesurées (mPa.s) | 230000 | 22000 | 18000 |
| Stabilités 24h | OUI | NON - Déphasage | NON - Déphasage |

### • Auto-évaluations in vivo

Le produit de l'exemple 1 de l'invention a été évalué par un panel dans l'absolu, selon un test en aveugle, pour une durée d'utilisation d'une semaine selon les instructions d'utilisation fournies. Le panel était constitué de 65 femmes âgées de 20 à 50 ans, réparties équitablement, utilisatrices régulières d'un fond de teint de l'art antérieur correspondant à celui décrit dans la fiche MINTEL N°10174267.

Voici la synthèse des appréciations qui ont été recueillies. Il a été vérifié que la moyenne des valeurs numériques obtenues est significative.

### APPRÉCIATION GLOBALE

- Très bonne satisfaction : « satisfaites » à 98%
- Très bonnes intentions d'achat : achat à 94%
- Préférence pour le produit testé : 60%, Préférence pour le produit habituel : 15%, et sans préférence 25%.

### APPRÉCIATION EN SPONTANÉ :

Spontanément, toutes les femmes ont émis au moins un commentaire positif vis-à-vis du fond de teint et les critiques sont mineures. Le produit est apprécié pour :
- son maquillage : résultat uniforme, naturel, beau/joli, couvrant, lumineux, qui tient bien
- son fini peau : pas d'impression de lourdeur, peau douce, confortable et surtout non brillant
- sa texture : très légère, agréable, souple et douce
- son application très facile

### TEXTURE :

Le produit présente une cosmétique très appréciée :
- une texture agréable (100%, 89% "très agréable"),
- une texture douce (100%, 86% "très douce"),
- une texture légère (100%, 71% "très légère"), fine (98%, 57% "très fine"), avec une consistance très bien équilibrée (91% "Bien comme il faut")

### APPLICATION:

Une application très facile (97%, 85% "très facilement"), très rapide (97%, 79% "très rapidement"), très homogène (97%, 71% "très homogène"), sans démarcations (98%), ni accumulation dans les reliefs de la peau (97%) ou trainées (95%) et sans aucune peluche (100%)

### RESULTAT MAQUILLAGE:

Très bon résultat maquillage :
- Une peau ni brillante (A l'application : 98%; Quelque temps après 95%), ni collante (100% A l'application et Quelques temps après) et qui reste très confortable tout au long de la journée (100% dont 75% "très confortable")
- Un teint très bien unifié (Top2 : 98%, 78% "très bien") avec une bonne couvrance (86% "Bien comme il faut"), un fini à la fois mat (48%) et poudré (41%) au rendu plutôt naturel (59%) et ayant une très bonne tenue (∼9h au global; ∼8h pour la matité).

### PROFIL D'IMAGE:

Avant tout, le film maquillage est fin, sans effet masque ni matière, le teint est matifié, parfaitement unifié, la peau paraît plus belle, plus lisse, les zones de sècheresse ne sont pas marquées et la couleur ne vire pas, elle reste homogène ("Tout à fait d'accord" >60%).

Le teint est également sublimé, lumineux, "parfait", bien corrigé, les pores sont moins visibles, le grain de peau est affiné, le maquillage est mat mais éclatant et le fond de teint tient bien et la matité est durable ("Tout à fait d'accord" ∼55%-45%).

En conclusion, le produit de l'invention permet une application très légère combinée à une couvrance élevée qui masque efficacement les imperfections de la peau, tout en offrant un confort à l'application très appréciable et qui perdure dans le temps.

## Revendications

1. Composition cosmétique de fond de teint pour le soin et le maquillage de la peau sous la forme d'une mousse **caractérisée par** une viscosité à 25°C et pression atmosphérique comprise entre 50 000 mPa.s et 500 000 mPa.s, et une densité à 25°C et pression atmosphérique comprise entre 0,8 g/cm³ et 1,2 g/cm³, ladite composition comprenant :
- de 35% à 75% d'au moins une huile volatile,
- de 1% à 6% d'au moins un polymère de silicone réticulé obtenu par réaction d'un premier organopolysiloxane comprenant au moins un groupe -Si-H en position terminale avec un deuxième organopolysiloxane comprenant au moins deux groupements vinyle, le polymère de silicone réticule étant un polysilicone-11 sous forme gélifiée,
- de 0,5% à 5% d'une argile organomodifiée qui est une hectorite organomodifiée modifiée avec un chlorure d'alkylammonium quaternaire, et
- moins de 5% d'eau,
- moins de 1% de composé(s) gras solide(s),
le ratio massique entre le polysilicone-11 et l'hectorite organomodifiée étant compris entre 1/1 et 10/1, et les pourcentages étant exprimés en masse par rapport à la masse de ladite composition.

2. Composition selon la revendication 1, **caractérisée en ce qu'**elle comprend de 1% à 10% en masse d'une résine siliconée par rapport à la masse de ladite composition.

3. Composition selon l'une des revendications précédentes, **caractérisée en ce qu'**une huile volatile est l'isododécane.

4. Composition selon l'une des revendications précédentes, **caractérisée en ce qu'**elle comprend de 10% à 30% en masse de particules solides.

5. Procédé de soin ou de maquillage de la peau qui consiste à appliquer sur la peau du visage, une composition selon l'une des revendications 1 à 4.

## Patentansprüche

1. Kosmetische Grundierungszusammensetzung für die Pflege und das Make-up der Haut in Form eines Schaums, **gekennzeichnet durch** eine Viskosität zwischen 50.000 mPa.s und 500.000 mPa.s bei 25 °C und Atmosphärendruck und eine Dichte zwischen 0,8 g/cm³ und 1,2 g/cm³ bei 25 °C und Atmosphärendruck, wobei die Zusammensetzung umfasst:
- 35 % bis 75 % mindestens eines flüchtigen Öls,
- 1 % bis 6 % mindestens eines vernetzten Siliconpolymers, das durch Reaktion eines ersten Organopolysiloxans, das mindestens eine -Si-H-Gruppe in Endstellung umfasst, mit einem zweiten Organopolysiloxan, das mindestens zwei Vinylgruppen umfasst, erhalten wird, wobei es sich bei dem vernetzten Siliconpolymer um ein Polysilicon-11 in gelierter Form handelt,
- 0,5 % bis 5 % eines organomodifizierten Tons, bei dem es sich um einen mit einem quaternären Alkylammoniumchlorid modifizierten organomodifizierten Hectorit handelt, und
- weniger als 5 % Wasser,
- weniger als 1 % feste Fettverbindung (en),
wobei das Massenverhältnis zwischen dem Polysilicon-11 und dem organomodifizierten Hectorit zwischen 1/1 und 10/1 liegt und die Prozentsätze als Massenanteile, bezogen auf die Masse der Zusammensetzung, ausgedrückt sind.

2. Zusammensetzung nach Anspruch 1, **dadurch gekennzeichnet, dass** sie 1 bis 10 % Massenanteil eines Siliconharzes, bezogen auf die Masse der genannten Zusammensetzung, umfasst.

3. Zusammensetzung nach einem der vorhergehenden Ansprüche, **dadurch gekennzeichnet, dass** es sich bei einem flüchtigen Öl um Isododecan handelt.

4. Zusammensetzung nach einem der vorhergehenden Ansprüche, **dadurch gekennzeichnet, dass** sie 10 bis 30 % Massenanteil an festen Teilchen umfasst.

5. Verfahren zur Pflege oder zum Make-up der Haut, das darin besteht, auf die Haut des Gesichts eine Zusammensetzung nach einem der Ansprüche 1 bis 4 aufzutragen.

## Claims

1. A cosmetic foundation composition for caring for and making up the skin, in the form of a foam **characterized in that** its viscosity at 25°C and atmospheric pressure is between 50 000 and 500 000 mPa.s, and its density at 25°C and atmospheric pressure is between 0.8 and 1.2 g/cm³ , said composition comprising:
- from 35% to 75% of at least one volatile oil,
- from 1% to 6% of at least one crosslinked silicone polymer obtained by reaction of a first organopolysiloxane comprising at least one -Si-H group in an end position with a second organopolysiloxane comprising at least two vinyl groups, said crosslinked silicone polymer being a polysilicone-11 in gelified form,
- from 0.5% to 5% of one organomodified clay which is an hectorite modified with a quaternary alkylammonium chloride,
- less than 5% of water,
- less than 1% of solid fatty compound(s),
the mass ratio between the polysilicone-11 and the organomodified hectorite being between 1/1 and 10/1, and the percentages being expressed on a mass basis relative to the mass of said composition.

2. The composition as claimed in claim 1, **characterized in that** it comprises from 1% to 10% by mass of a silicone resin relative to the mass of said composition.

3. The composition as claimed in any one of the preceding claims, **characterized in that** the volatile oil is isododecane.

4. The composition as claimed in any one of the preceding claims, **characterized in that** it comprises from 10% to 30% by mass of solid particles.

5. A process for caring for or making up the skin, which consists in applying to facial skin a composition as claimed in any one of claims 1 to 4.
